Europäisches Patentamt

European Patent Office

Office européen des brevets

(11) Publication number: **0 134 215**
**B1**

(12) **EUROPEAN PATENT SPECIFICATION**

(45) Date of publication of patent specification: **25.10.89**

(51) Int. Cl.⁴: **G 01 N 33/543, G 01 N 33/74**

(21) Application number: **83902817.2**

(22) Date of filing: **26.08.83**

(86) International application number:
**PCT/GB83/00210**

(87) International publication number:
**WO 84/01031 15.03.84 Gazette 84/07**

(54) MEASUREMENT OF ANALYTE CONCENTRATION.

(30) Priority: **27.08.82 GB 8224600**

(43) Date of publication of application:
**20.03.85 Bulletin 85/12**

(45) Publication of the grant of the patent:
**25.10.89 Bulletin 89/43**

(84) Designated Contracting States:
**DE FR GB**

(56) References cited:
**EP-A-0 015 687**
**EP-A-0 026 103**
**WO-A-82/01773**
**GB-A-2 085 160**

(73) Proprietor: **EKINS, Roger Philip**
**Department of Molecular Endocrinology**
**University College & Middlesex School of**
**Medicine Mortimer Street**
**London W1N 8AA (GB)**

(72) Inventor: **EKINS, Roger Philip**
**Department of Molecular Endocrinology**
**University College & Middlesex School of**
**Medicine Mortimer Street**
**London W1N 8AA (GB)**

(74) Representative: **Hale, Stephen Geoffrey et al**
**J.Y. & G.W. Johnson Furnival House 14/18 High**
**Holborn**
**London WC1V 6DE (GB)**

Courier Press, Leamington Spa, England.

## Description

### Technical field

The present invention relates to the measurement of ambient analyte concentrations in fluids, primarily the concentrations of hormones and other biologically active substances in body fluids such as saliva, serum, blood and urine.

### Background art

It is known (for example from EP—A—15687) to measure the concentration of a free hormone in a body fluid also containing that hormone in endogenously (reversibly) bound form by contacting a fluid sample of accurately determined volume with a binding agent having binding sites specific for the free hormone, usually an antibody, and with a radioactively labelled form of the free hormone. The binding agent binds a proportion of the unlabelled free hormone and a proportion of the labelled free hormone, the relative amount of labelled hormone bound being a function of the amount of unlabelled hormone present in the sample. The results obtained are calibrated by comparison with the results obtained with standard solutions containing known concentrations of unlabelled free hormone, and thus the actual amount of unlabelled free hormone in the unknown sample is determined.

Important disadvantages of the known technique are that a sample of the body fluid in question has to be removed from the body and placed in a test tube or the like and its absolute volume needs to be known. It would be an advantage to avoid one or both of these difficulties.

### Disclosure of invention

The present invention provides a method of measuring ambient analyte concentration in a fluid by contacting the fluid with a binding agent having binding sites specific for the analyte as compared with the other components of the fluid, determining a figure representative of the extent of binding of the analyte to the binding agent and estimating therefrom the analyte concentration in the fluid, characterised in that the binding agent is used in a trace amount which has at most an insignificant effect on the total concentration of free analyte in the fluid, and a figure representative of the proportional occupancy of the binding sites on the binding agent is determined and used to estimate the analyte concentration in the fluid, without estimating as an essential intermediate step the total amount of analyte in the fluid contacted with the binding agent, so that it is not necessary to measure the volume of the fluid contacted with the binding agent.

According to the invention means are provided whereby the ambient concentration of an analyte, such as a hormone, in a fluid can be measured without the need to know the volume of the fluid being measured and hence, in the case of body fluids, without the need to remove the body fluid from the body.

This invention is based on the fact, not hitherto appreciated, that when a fluid containing an analyte such as a hormone is contacted with an antibody or other binding agent having binding sites specific for the analyte, the occupancy of the binding sites by the analyte (proportion of binding sites occupied) is independent of the absolute volume of the fluid and the absolute number of binding sites, and hence independent of the absolute amount of binding agent, provided only that the relative amount of analyte and binding agent and the affinity between them are such that the introduction of the binding agent into the fluid has no significant effect on the concentration of the analyte. Thus, for example, if only a trace amount of binding agent is used, such that only an insignificant fraction of the analyte becomes bound to the binding agent, then the overall analyte concentration in the fluid will not change noticeably.

Provided that the above condition holds, the concentration [H] of analyte in the fluid is related to the fraction of binding sites occupied ($Ab/Ab_o$) by the equation

$$\frac{Ab}{Ab_o} = \frac{K_{ab}[H]}{1+K_{ab}[H]}$$

where $K_{ab}$ is the equilibrium constant for the binding of the analyte to the binding sites and is a constant for the given analyte and binding agent at a given temperature.

A close analogy to this basic idea is provided by the use of a simple thermometer for the measurement of ambient temperatures. The introduction of a thermometer into—for example—a room generally implies uptake of heat by the thermometer and hence a (usually insignificant) disturbance to the pre-existing temperature of the room. Provided the thermal capacity of the room and its contents are large as compared with that of the thermometer, the temperature ultimately recorded by the thermometer essentially reflects the original room temperature. Likewise the binding-site occupancy of an antibody or other binding agent probe introduced into a biological or other fluid will, assuming the conditions mentioned above are adhered to, reflect the analyte concentration originally present in the fluid.

Accordingly it is possible to design a probe which contains immobilised binding agent in low concentrations, to insert this into the fluid whose ambient analyte concentration is to be measured and, either once equilibrium has been reached or before equilibrium is reached, to determine the proportion of antibody sites occupied by the analyte. Such a determination will frequently be performed on the probe after withdrawal from the fluid, although this is not an essential feature of the invention, and it would be advantageous in some cases to make the determination in situ and perhaps to couple it with feed-back measures to correct any imbalance of analyte concentration detected, for example to maintain a particular hormone level in a body fluid.

Therefore, the present invention provides in broad terms in one aspect a method of measuring ambient analyte concentration in a fluid, the method comprising contacting an unmeasured volume of the fluid with a trace amount of binding agent having binding sites specific for the analyte and estimating from the proportional occupany of the binding sites the concentration of analyte in the fluid. In this context the term "trace" denotes an amount which has only an insignificant effect on the total concentration of free analyte in the fluid.

The method may be used for the estimation of analytes of all types provided that a specific binding agent is available. However, it is likely to be of greatest value in the estimation of biologically active materials such as drugs, viruses and particularly hormones, where other estimation methods are more complex. The analytes may be present in any fluid from simple aqueous solutions up to biological fluids of all types but estimation of concentrations in body fluids provides a particularly important area. The presence or absence of other ingredients is immaterial provided that they do not interfere with the binding of the analyte. The hormones may be present in fluids also containing endogenously bound hormone but this need not be the case.

A wide variety of binding agents may also be used provided that they have binding sites which are specific for the analyte in question, as compared with any other ingredient in the fluid in question. When estimating concentrations of hormones or other naturally occurring body chemicals it may be advantageous to use antibodies for the chemical in question where these are readily obtainable. However, other binding agents such as binding proteins or receptor preparations (preparations containing receptor sites and derived from areas of the body where the chemical in question normally becomes bound) may also be used.

Conveniently, the binding agent used will be immobilised on a solid support (although soluble binding agents could be used and later precipitated or otherwise separated to enable the binding site occupancy to be estimated). The solid supports used may be those which are conventional for this purpose, including cellulose and polysaccharide such as Sephadex (Registered Trade Mark). When, according to a preferred embodiment of the invention, the concentration of an analyte in a body fluid is to be estimated without removing the body fluid from the body, the support may be in any form convenient for insertion into an appropriate part of the body, for example a probe made of polystyrene of other rigid non-harmful plastics material.

Preferably the binding agent chosen will be one whose equilibrium constant $K_{ab}$ in the above equation is such that the proportion of binding sites occupied by the analyte at its expected concentration in the fluid will be considerably less than 100%, more preferably less than 75%. This gives greater sensitivity to variations in concentration. The binding agent chosen will therefore normally be different in its thermodynamic characteristics from the antibody chosen for use in known radioimmunoassay determinations of hormone concentration, where it is normally desirable to have as high an occupancy of binding sites on the antibody, and hence as high an equilibrium constant, as possible.

When the occupancy of binding sites on the binding agent is to be determined by methods involving binding the unoccupied sites with a reagent whose presence is discernible, for example a radioactively labelled form of the analyte, the equilibrium constant $K_{ab}$ is preferably such that a substantial proportion of the binding sites are occupied, advantageously at least 25%, because this gives greater sensitivity in the final measurement. Under such circumstances, the equilibrium constant of the binding agent is preferably close to the reciprocal of the expected analyte concentration because this will lead to a binding site occupancy close to 50%.

The nature of the method of estimating the occupancy of binding sites on the binding protein is not an essential part of the present invention in its broadest form and a variety of methods can be used. The simplest of these is a back-titration of unoccupied sites by the use of a labelled reagent which binds with unoccupied sites but it is also possible to use a sandwich-type or two-site approach. Alternatively, the extent of occupancy can be measured by biochemical or other means in situ.

Where back-titration assay methods are being used, it is preferred to use a binding agent whose dissociation constant for the uncoupling of analyte is low in order to avoid measurement errors as a result of premature dissociation of the analyte from the binding agent. The rate at which equilibrium is reached may also be slow, although if sufficiently small amounts of binding agent are used relative to analyte the equilibrium can be expected to be reached relatively quickly. It is also possible to make measurements before equilibrium is reached and to deduce from them the concentrations involved, but is adds to the complexity of the operation and reduces the accuracy and consequently it is not recommended.

With the use of small amounts of binding agent for the test methods it becomes of greater importance to have a labelled reagent of high specific activity for the backtitration to determine the proportion of unoccupied binding sites because, in general, only small absolute numbers of occupied and unoccupied binding sites will be present. Accordingly, instead of conventional radioisotopic labels it may be desirable to employ labels of other types such as fluorescent labels.

An added advantage of the use of fluorescent labels or others of very high specific activity for analyte-labelling is that they make possible the development of very high sensitivity, multiple-analyte, assays relying on the scanning of the distribution of fluorescent labels (comprising labelled antibodies and/or labelled analytes) deposited on the surface of—for example—a suitable plastics material. Such a surface—"printed" with a mixture of different antibodies and subsequently exposed to the biological fluid under test—can potentially be used to reveal the concentrations of many different analytes in the same sample—a requirement which is likely to become increasingly pressing in the monitoring of blood for the presence of complex mixtures of viral antigens and/or antibodies, tumour antigens and hormones.

The concept of the "immunometer"—i.e. the analyte concentration sensing device discussed in the preceding paragraph—is likely to bring about significant changes in research and of routine clinical diagnosis. For example, steroid and thyroid hormone levels may ultimately be monitored—not by analysis of blood samples as is current normal practice—but by examination of the antibody-binding site occupancy of plastic probes following their insertion, for a few minutes, into the subject's mouth, and their exposure to ambient hormone levels present in the saliva.

The following Examples illustrate the basis for the invention.

## Example 1

Antibody directed against a hormone occurring in a body fluid is diluted in a 0.05M barbital buffer at pH 8.7 and the resulting fluid is exposed to a probe in the form of a plastics support made of polystyrene for 2 to 16 hours at ambient temperature. The plastics support is then removed and thoroughly washed and can then be used as a probe to estimate the concentration of the hormone in appropriate fluids by the method of the invention after its affinity (equilibrium constant) and binding capacity have been assessed by a known method.

## Example 2

Antibody directed against hydrocortisone (cortisol), obtained from the Tenovirus Institute for Cancer Research, Welsh National School of Medicine, Cardiff, Wales, was coupled to a solid support and the equilibrium constant ($K_{ab}$) and binding capacity of the resulting material were measured, by Scatchard analysis, and found to be $2\times10^{10}$ litres/mole and 100 pmoles/ml respectively.

Standard cortisol solutions containing cortisol concentrations of 100 pM, 1 nM, 10nM, 100nM and 1μM were prepared by dissolving pure cortisol (H4001, from Sigma Chemical Co., Poole, England) in a buffer solution of 0.05M $KH_2PO_4$ and 0.15M NaCl (pH 7.4) (hereinafter referred to as PBS) containing 0.1% by weight gelatine (No. 44045, from BDH Chemicals Ltd., Poole, England). Amounts of the antibody preparation having a binding capacity of less than 10 fmoles of cortisol were incubated to equilibrium at 20°C (16 hours, although equilibrium had for practical purposes been achieved within 20 minutes) with samples of each of the standard cortisol solutions having volumes of 0.2, 0.4 and 0.8 ml. After incubation the samples were cooled, on ice, to 4°C and the solid material washed thoroughly with PBS.

The extent of occupancy of the antibody binding sites by cortisol was determined in each case by a radioimmunoassay back-titration using as the labelled material a high specific activity iodinated cortisol ($\sim3.7\times10^{13}$Bq/mmole [125]I), obtained from RIA Ltd., Cardiff, Wales.

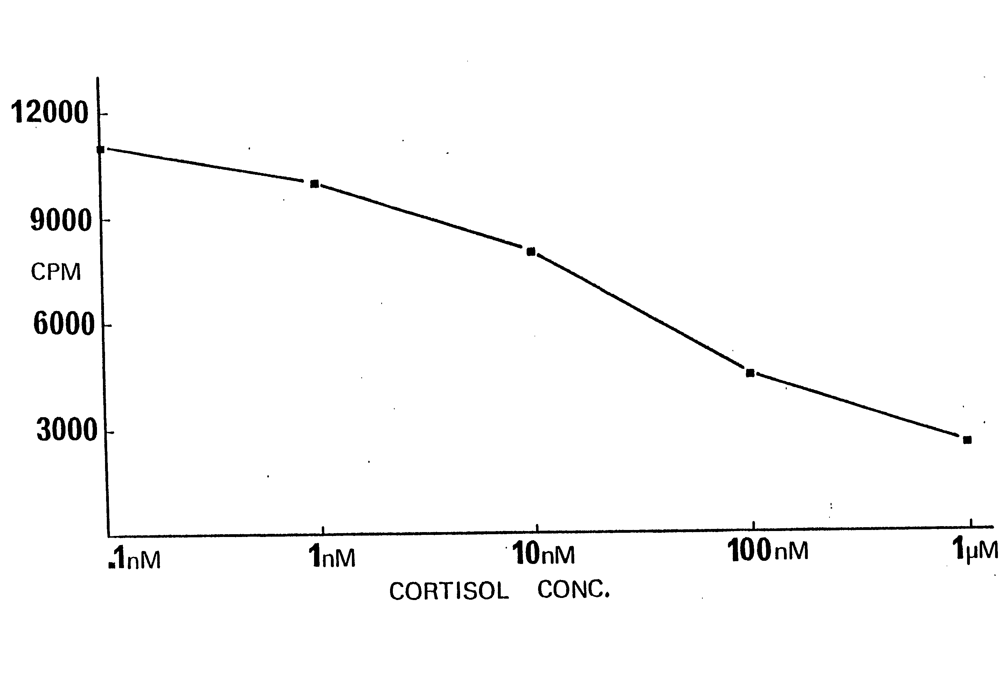

Concentrated [125]I-cortisol was added and mixed with the solid material in each sample and incubation was continued for 1 hour at 4°C. The solid material was again thoroughly washed and the bound radioactivity determined. The accompanying drawing is a graph showing the relationship between the observed radioactivity (in counts per minute) and the cortisol concentration (in nM) of the samples of the standard solutions. Within the limits of experimental error, the level of bound radioactivity was the same for all three samples of identical cortisol concentration and was unaffected by their differences in volume.

## Claims

1. A method of measuring ambient analyte concentration in a fluid by contacting the fluid with a binding agent having binding sites specific for the analyte as compared with the other components of the fluid, determining a figure representative of the extent of binding of the analyte to the binding agent and estimating therefrom the analyte concentration in the fluid, characterised in that the binding agent is used in a trace amount which has at most an insignificant effect on the total concentration of free analyte in the fluid, and a figure representative of the proportional occupancy of the binding sites on the binding agent is determined and used to estimate the analyte concentration in the fluid, without estimating as an essential intermediate step the total amount of analyte in the fluid contacted with the binding agent, so that it is not necessary to measure the volume of the fluid contacted with the binding agent.

2. A method as claimed in claim 1, characterised in that the analyte is a biologically active material and the binding agent is an antibody for the analyte.

3. A method as claimed in claim 2, characterised in that the biologically active material is a hormone, for example cortisol and it is not the case that the fluid also contains the hormone in endogenously reversibly bound form.

4. A method as claimed in any of claims 1 to 3, characterised in that the volume of the fluid contacted with the binding agent is not measured accurately.

5. A method as claimed in any of claims 1 to 4, characterised in that the binding agent is contacted with the fluid whilst immobilised on a solid support, and the binding agent and solid support are separated from the fluid before the figure representative of the proportional occupancy of the binding sites is determined.

6. A method as claimed in claim 5, characterised in that the figure representative of the proportional occupancy of the binding sites on the binding agent is determined by back-titration using a fluorescent labelled reagent.

7. A method as claimed in any of claims 1 to 6, characterised in that the fluid is a body fluid such as saliva, serum, blood or urine, and the binding agent is immobilised on a probe capable of being inserted into and withdrawn from the body fluid.

8. A method as claimed in any of claims 1 to 7, characterised in that a binding agent is chosen whose equilibrium constant for the binding of the analyte to the binding sites is such that less than 75% of the binding sites of the binding agent will be occupied by the analyte at its expected concentration in the fluid.

9. A method as claimed in claim 8, characterised in that a binding agent is chosen whose equilibrium constant for the binding of the analyte to the binding sites is such that more than 25% of the binding sites of the binding agent will be occupied by the analyte at its expected concentration in the fluid and the figure representative of the proportional occupancy of the binding sites is determined by a method involving binding the unoccupied sites with a reagent whose presence is discernible.

10. A device for measuring the concentration of an analyte in a body fluid of a living creature without removing a sample of the body fluid from the living creature, characterised by a solid probe designed to the introduced into and withdrawn from the body fluid and having immobilised thereon a binding agent having binding sites specific for the analyte as compared to the other components of the body fluid, the binding agent being present in a trace amount such that the insertion of the probe into the body fluid has at most an insignificant effect on the total concentration of the free analyte in the body fluid.

**Patentansprüche**

1. Verfahren zur Messung der in einer Flüssigkeit vorliegenden Analytkonzentration, bei dem man die Flüssigkeit mit einem bindungsfähigen Stoff in Berührung bringt, der für den Analyten gegenüber den übrigen Komponenten der Flüssigkeit spezifische Bindungsstellen aufweist, eine das Ausmaß der Bindung des Analyten an den bindungsfähigen Stoff darstellende Größe bestimmt und daraus die Analytkonzentration in der Flüssigkeit ermittelt, dadurch gekennzeichnet, daß man den bindungsfähigen Stoff in einer Spurmenge einsetzt, die einen höchstens unbedeutenden Einfluß auf die Gesamtkonzentration an freiem Analyten in der Flüssigkeit ausübt, eine die proportionale Besetzung der Bindungsstellen auf dem bindungsfähigen Stoff darstellende Größe bestimmt und diese dazu benutzt, die Analytkonzentration in der Flüssigkeit zu ermitteln, ohne die Gesamtmenge an Analyt in der mit dem bindungsfähigen Stoff in Berührung gebrachten Flüssigkeit als unbedingt notwendige Zwischenstufe zu ermitteln, so daß das Volumen der mit dem bindungsfähigen Stoff in Berührung gebrachten Flüssigkeit nicht gemessen zu werden braucht.

2. Verfahren nach Anspruch 1, dadurch gekennzeichnet, daß der Analyt ein biologisch aktiver Stoff und der bindungsfähige Stoff ein Antikörper für den Analyten ist.

3. Verfahren nach Anspruch 2, dadurch gekennzeichnet, daß der biologisch aktive Stoff ein Hormon ist, zum Beispiel Cortisol, und das Hormon dabei nicht auch in endogen (reversibel) gebundener Form in der Flüssigkeit vorliegt.

4. Verfahren nach einem der Ansprüche 1 bis 3, dadurch gekennzeichnet, daß das Volumen der mit dem bindungsfähigen Stoff in Berührung gebrachen Flüssigkeit nicht genau gemessen wird.

5. Verfahren nach einem der Ansprüche 1 bis 4, dadurch gekennzeichnet, daß der bindungsfähige Stoff mit der Flüssigkeit in Berührung gebracht wird, während dieser auf einem festen Träger immobilisiert ist, und der bindungsfähige Stoff und der feste Träger vor der Bestimmung der die proportionale Besetzung der Bindungsstellen darstellenden Größe von der Flüssigkeit abgetrennt werden.

6. Verfahren nach Anspruch 5, dadurch gekennzeichnet, daß die die proportionale Besetzung der Bindungsstellen auf dem bindungsfähige Stoff darstellende Größe durch Rücktitration mit einem fluoreszenzmarkierten Reagenz bestimmt wird.

7. Verfahren nach einem der Ansprüche 1 bis 6, dadurch gekennzeichnet, daß als Flüssigkeit eine Körperflüssigkeit wie Speichel, Serum, Blut oder Urin vorliegt und der bindungsfähige Stoff auf einer Sonde immobilisiert ist, die in die Körperflüssigkeit eingeführt und daraus zurückgezogen werden kann.

8. Verfahren nach einem der Ansprüche 1 bis 7, dadurch gekennzeichnet, daß man einen bindungsfähigen Stoff auswählt, dessen Gleichgewichtskonstante für die Bindung des Analyten an die Bindungsstellen so liegt, daß bei der erwarteten Konzentration des Analyten in der Flüssigkeit dieser weniger als 75% der Bindungsstellen auf dem bindungsfähigen Stoff besetzen wird.

9. Verfahren nach Anspruch 8, dadurch gekennzeichnet, daß man einen bindungsfähigen Stoff auswählt, dessen Gleichgewichtskonstante für die Bindung des Analyten an die Bindungsstellen so liegt, daß bei der erwarteten Konzentration des Analyten in der Flüssigkeit dieser mehr als 25% der Bindungsstellen auf dem bindungsfähigen Stoff besetzen wird, und daß man die die proportionale Besetzung der Bindungsstellen darstellende Größe nach einer Methode ermittelt, bei der es sich um eine Bindung der unbesetzten Stellen mit einem Reagenz handelt, dessen Gegenwart nachweisbar ist.

10. Vorrichtung zur Messung der Konzentration eines Analyten in einer Körperflüssigkeit eines Lebewesens ohne eine Probe der Körperflüssigkeit aus diesem zu ziehen, gekennzeichnet durch eine feste Sonde, die zur Einführung in die Körperflüssigkeit und Zurückziehung daraus ausgebildet ist und darauf immobilisiert einen bindungsfähigen Stoff mit für den Analyten gegenüber den übrigen Komponenten der Körperflüssigkeit spezifischen Bindungsstellen aufweist, wobei der bindungsfähige Stoff in einer solchen Spurenmenge vorliegt, daß die Einführung der Sonde in die Körperflüssigkeit einen höchstens unbedeutenden Einfluß auf die Gesamtkonzentration an freiem Analyten der Körperflüssigkeit ausübt.

**Revendications**

1. Une méthode de mesure de la concentration ambiante en substance à analyser dans un fluide par mise en contact du fluide avec un agent liant ayant des sites de liaison spécifiques de la substance à analyser par rapport aux autres composants du fluide, la détermination d'un chiffre représentatif du taux de liaison de la substance à analyser à l'agent liant et l'estimation à partir de celui-ci de la concentration en substance à analyser du fluide, caractérisé en ce que l'un utilise l'agent liant en quantité de traces qui ont tout au plus un effet insignifiant sur la concentration totale en substance à analyser libre du fluide, et en ce que l'on détermine un chiffre représentatif du niveau proportionnel d'occupation des sites de liaison sur l'agent liant et en ce que l'on utilise ce chiffre pour estimer la concentration en substance à analyser du fluide, sans estimer en tant qu'étape intermédiaire essentielle la quantité totale de substance à analyser dans le fluide mis en contact avec l'agent liant, de manière qu'il ne soit pas nécessaire de mesurer le volume de fluide mis en contact avec l'agent liant.

2. Une méthode selon la revendication 1, caractérisée en ce que la substance à analyser est une substance biologiquement active et en ce que l'agent liant est un anticorps pour la substance à analyser.

3. Une méthode selon la revendication 2, caractérisé en ce que la substance biologiquement active est une hormone, par exemple le cortisol, et en ce que l'on ne se trouve pas dans le cas où le fluide contient également l'hormone sous une forme liée de manière endogène (réversible).

4. Une méthode selon l'une quelconque des revendications 1 à 3, caractérisée en ce que l'on ne mesure pas avec précision le volume de fluide mis en contact avec l'agent liant.

5. Une méthode selon l'une quelconque des revendications 1 à 4, caractérisée en ce que l'on met en contact l'agent liant avec le fluide alors qu'il est immobilisé sur un support solide, et en ce que l'on sépare l'agent liant et le support solide du fluide avant de déterminer le chiffre représentatif du niveau proportionnel d'occupation des sites de liaison.

6. Une méthode selon la revendication 5, caractérisée en ce que l'on détermine le chiffre représentatif du niveau proportionnel d'occupation des sites de liaison sur l'agent liant par titrage en retour au moyen d'un réactif fluorescent marqué.

7. Une méthode selon l'une quelconque des revendications 1 à 6, caractérisée en ce que le fluide est un fluide corporel, tel que de la salive, du sérum, du sang ou de l'urine et en ce que l'on immobilisée l'agent liant sur une sonde que l'on peut insérer dans le fluide corporel et l'en retirer.

8. Une méthode selon l'une quelconque des revendications 1 à 7, caractérisée en ce que l'on choisit un agent liant dont la constante d'équilibre pour la liaison de la substance à analyser aux sites de liaison est telle que moins de 75% des sites de liaison de l'agent liant seront occupés par la substance à analyser à sa concentration attendue dans le fluide.

9. Une méthode selon la revendication 8, caractérisée en ce que l'on choisit un agent liant dont la constante d'équilibre pour la liaison de la substance à analyser aux sites de liaison est telle que plus de 25% des sites de liaison de l'agent liant seront occupés par la substance à analyser à sa concentration attendue dans le fluide, et en ce que l'on détermine le chiffre représentatif du niveau proportionnel d'occupation des sites de liaison par une méthode qui implique la liaison des sites inoccupés à l'aide d'un réactif dont on peut discerner la présence.

10. Un dispositif pour mesurer la concentration d'une substance à analyser dans un fluide corporel d'une créature vivante sans retirer un échantillon de son fluide corporel, caractérisé par une sonde solide conçue pour être introduite dans le fluide corporel et pour en être retirée, et possédant immobilisé sur elle un agent liant ayant des sites de liaison spécifiques de la substance à analyser par rapport aux autres composants du fluide corporel, l'agent liang étant présent en quantité de traces telles que l'insertion de la sonde dans le fluide corporel a tout au plus un effet insignifiant sur la concentration totale de la substance à analyser libre dans le fluide corporel.

EP  0 134 215  B1

CORTISOL  CONC.

12000  9000  CPM  6000  3000

1nM   1nM   10nM   100nM   1μM